# DEMANDE DE BREVET EUROPEEN

(11) **EP 4 670 834 A1**
(43) Date de publication de la demande: **31.12.2025**
(21) Numéro de dépôt: 25184890.9
(22) Date de dépôt: 24.06.2025
(51) Int. Cl.: B01J 13/18, A01N 25/28, A61K 8/11, A61K 8/25, A61Q 19/00, B01J 13/22, C11D 3/50

(54) **PROCÉDÉ DE PRÉPARATION DE MICROCAPSULES D'ACTIFS LIPOPHILES DANS DES MATÉRIAUX INORGANIQUES ET INORGANIQUES HYBRIDES**

(30) Priorité: 25.06.2024 FR 2406808
(71) Demandeur: Lifescientis, 06130 Grasse (FR)
(72) Inventeur: Avdienko, Anastasia, 06400 Cannes (FR); Saldo, Julien, 06140 Vence (FR); Fabio, Karine, 06610 La Gaude (FR); Chuzel, Franck, 06370 Mouans Sartoux (FR)
(74) Mandataire: Hautier IP

(57) **Abrégé**

L'invention concerne un procédé de synthèse de microcapsules de silice comprenant une phase huileuse comprenant au moins un actif lipophile comprenant les étapes suivantes :
a) préparation de nucléi de silice par hydrolyse d'au moins un précurseur de silice dans l'eau en milieu catalytique acide, a') ajout à l'issu de l'étape a) ou au cours de l'étape a) d'une phase huileuse dans un ratio phase huileuse / précurseur de silice supérieur ou égal à 1/1
b) formation d'une phase matrice par mélange d'au moins un agent de condensation choisi parmi au moins un polymère polycationique basique branché ou linéaire, et d'au moins un anion monovalent, divalent ou trivalent,
c) condensation en milieu basique des nucléis de silice obtenus à l'étape a) et de la phase huileuse par mélange sous agitation avec la phase matrice obtenue à l'étape b) à pH alcalin inférieur ou égal à 10.

## Description

### DOMAINE TECHNIQUE

La présente invention concerne le domaine de la synthèse par chimie douce et plus particulièrement pour la préparation de microcapsules d'actifs lipophiles entourés de matériaux inorganiques et/ou de matériaux inorganiques hybrides. Les microcapsules formées sont destinées à être utilisées en tant que système d'emport d'actifs. Les domaines d'application sont les cosmétiques, la parfumerie, le soin du linge/de la maison, les produits d'hygiène et de santé, les biocides, les biostimulants, les produits phytopharmaceutiques, agroalimentaires et les répulsifs à usage humain ou vétérinaire.

### ETAT DE LA TECHNIQUE

La microencapsulation est une technologie permettant l'immobilisation d'un ingrédient actif dans une microcapsule présentant une taille de 1µm à 1000 µm. L'ingrédient actif est finement dispersé dans une matrice continue (sphère) ou enrobé d'une couche de matériau (capsule ou cœur/écorce).

La microencapsulation est utilisée pour stabiliser un actif, le protéger de phénomènes chimiques ou physiques d'oxydation, humidité, chaleur, rayonnements UV et contrôler sa libération dans le temps ou sous divers stimuli extérieurs (chaleur, frottement, pH). La microencapsulation peut apporter une forte valeur ajoutée et de nouvelles fonctionnalités aux ingrédients encapsulés et trouve ainsi de nombreuses applications industrielles notamment dans l'industrie pharmaceutique (humaine et vétérinaire), agroalimentaire, cosmétique (humaine et vétérinaire), phytosanitaire, des parfums et des arômes.

De nombreux procédés de microencapsulation sont disponibles et basés sur des méthodes mécaniques, chimiques ou physico-chimiques tels que l'atomisation (spray-drying, spray-coating), l'extrusion, le lit fluidisé, les fluides supercritiques, les microgels d'alginate, la coacervation, la polymérisation interfaciale et la chimie sol-gel.

Les nombreux avantages de la microencapsulation dans des capsules de silice ou hybrides ont été décrits notamment dans le document US10,099,194 B2 qui concerne des microcapsules sol-gel de silice.

Ces microcapsules sol-gel de silice sont généralement obtenues par hydrolyse et condensation d'un précurseur de silice en présence de solvant organique en milieu alcalin comme l'ammoniaque concentrée ou en milieu acide fort, de solvants hydrophobes ou de tensioactifs pétrosourcés. On connait notamment du document US 2012/104639 un procédé de synthèse de capsules de silices à partir une émulsion obtenue par des agents surfactants et une condensation sol-gel classique nécessitant une augmentation du pH par une base forte.

La chimie dite douce est de plus en plus recherchée pour développer des procédés de synthèse qui sont plus écologiques et qui s'intègrent de façon plus harmonieuse dans les processus naturels.

La demande de brevet FR3112494A1 du déposant propose un procédé de formation de silice par une méthode douce qui soit compatible avec une présence d'actif. Ce document ne cherche pas d'encapsulation d'une phase huileuse optimale.

Un objet de la présente invention est donc de proposer un procédé de synthèse de capsule de silice de taille contrôlée micronique, pouvant avantageusement permettre l'emport d'actifs lipophiles.

### RESUME

Pour atteindre cet objectif, selon un mode de réalisation on prévoit un procédé de synthèse de microcapsules de silice comprenant une phase huileuse comprenant au moins un actif lipophile comprenant les étapes suivantes :
a) préparation de nucléi de silice par hydrolyse d'au moins un précurseur de silice dans l'eau en milieu catalytique acide, préférentiellement la quantité d'acide est choisie de sorte à être inférieure ou égale à 0,05 équivalent d'acide par rapport au précurseur de silice,
a') ajout à l'issu de l'étape a) ou au cours de l'étape a) d'une phase huileuse dans un ratio en poids phase huileuse/ précurseur de silice supérieur ou égal à 1/1, préférentiellement inférieur ou égal à 7/1,
b) formation d'une phase matrice par mélange d'au moins un agent de condensation choisi parmi au moins un polymère polycationique basique branché ou linéaire choisi parmi l'un au moins parmi les polyéthylènes imines, les polyaminoacides, les polyallylamines, des dérivés des propylènes imines, les polylysines, les dérivés polysaccharides à galactomannanes, fructo-oligosaccharides et oligofructoses ou un mélange, et d'au moins un anion monovalent, divalent ou trivalent choisi parmi l'un au moins parmi un sel de phosphate, un sel de tartrate et un sel de citrate, un sel de sulfate, ou un sel de nitrate,
c) condensation en milieu basique des nucléis de silice obtenus à l'étape a) et de la phase huileuse par mélange sous agitation avec la phase matrice obtenue à l'étape b) à pH alcalin inférieur ou égal à 10.

Le présent procédé permet de manière surprenante d'améliorer la performance des microcapsules. L'invention permet notamment d'avoir un taux d'emport d'actifs lipophiles nettement supérieur à l'état de la technique, notamment par un ratio en poids actifs / précurseur de silice en faveur de l'actif dans une gamme sélectionnée. De plus, les microcapsules obtenues par le présent procédé présente une étanchéité optimisée ce qui permet notamment d'augmenter le degré de sécurité pour l'utilisateur en diminuant l'exposition de l'utilisateur à de potentiels toxiques et donc d'en contrôler la toxicité.

### BREVE DESCRIPTION DES FIGURES

Les buts, objets, ainsi que les caractéristiques et avantages de l'invention ressortiront mieux de la description détaillée d'un mode de réalisation de cette dernière qui est illustré par les dessins d'accompagnement suivants dans lesquels :
La figure 1 est un diagramme représentatif de la distribution granulométrique par volume obtenu par diffraction laser d'un échantillon de suspension de microcapsules de silice hybride contenant du CBD obtenues avec l'Exemple 1.
La figure 2 est une image représentative par microscopie optique de microcapsules de silice hybride contenant du CBD obtenues avec l'Exemple 1.
La figure 3 est une image représentative par microscopie électronique à balayage de microcapsules de silice hybride contenant du CBD obtenues avec l'Exemple 1.
Les figures 4A et 4B sont des images représentatives par microscopie optique à fluorescence (grossissement x20) d'échantillons de crème de jour contenant du CBD dans des microparticules de silice hybride obtenues avec l'exemple 1 avant (A) et après étalement (B) libérant le contenu en CBD.
Les figures 5A et 5B sont des chromatogrammes HPLC représentatifs d'échantillons de microparticules de silice hybride contenant du CBD obtenues avec l'exemple 1 après stockage 10j à 50°C (A) et à 4°C (B).
La figure 6 est un diagramme représentatif de la distribution granulométrique par volume obtenu par diffraction laser d'un échantillon de suspension de microcapsules de silice hybride contenant de l'HE Pélargonium obtenues avec l'Exemple 2.
La figure 7 est une image représentative par microscopie électronique à balayage de microcapsules de silice hybride contenant de l'HE Pélargonium obtenues avec l'Exemple 2.
Les figures 8A et 8B sont des chromatogrammes HPLC représentatifs d'un échantillon de microparticules de silice hybride contenant de l'HE Pélargonium obtenues avec l'Exemple 2 (A) et d'un échantillon d'HE Pélargonium (B).
La figure 9 est un diagramme représentatif de la distribution granulométrique par volume obtenu par diffraction laser d'un échantillon de suspension de microcapsules de silice hybride contenant de l'HE Orange Douce obtenues avec l'Exemple 3.
La figure 10 est une image représentative par microscopie optique de microcapsules de silice hybride contenant de l'HE Orange Douce obtenues avec l'Exemple 3.
Les figures 11A et 11B sont des chromatogrammes HPLC représentatifs d'un échantillon de microparticules de silice hybride contenant de l'HE Orange Douce obtenues avec l'Exemple 3 (A) et d'un échantillon d'HE Orange Douce (B).
La figure 12 est une image représentative par microscopie optique de microcapsules de silice hybride contenant de l'HE Orange Douce obtenues avec l'Exemple 4 et indiquant la présence d'HE Orange Douce non encapsulée.
La figure 13 est un diagramme représentatif de la distribution granulométrique par volume obtenu par diffraction laser d'un échantillon de suspension de microcapsules de silice hybride contenant une composition parfumée destinée au Personal Care obtenues avec l'Exemple 5.
La figure 14 est une image représentative par microscopie électronique à balayage de microcapsules de silice hybride contenant une composition parfumée destinée au Personal Care obtenues avec l'Exemple 5
La figure 15 est un diagramme représentatif de la distribution granulométrique par volume obtenu par diffraction laser d'un échantillon de suspension de microcapsules de silice hybride contenant une composition parfumée destinée au Personal Care obtenues avec l'Exemple 6.
La figure 16 est une image représentative par microscopie optique de microcapsules de silice hybride contenant contenant l'HE Pélargonium obtenues avec l'Exemple 8 sans l'utilisation de sels de phosphate ayant tendance à s'agréger par la présence de gel.
La figure 17 est un diagramme représentatif de la distribution granulométrique par volume obtenu par diffraction laser d'un échantillon de suspension de microcapsules de silice contenant une composition parfumée obtenues avec l'Exemple 9.
La figure 18 est une image représentative par microscopie optique de microcapsules de silice contenant une composition parfumée obtenues avec l'Exemple 9.
La figure 19 est un diagramme représentatif de la distribution granulométrique par volume obtenu par diffraction laser d'un échantillon de suspension de microcapsules de silice contenant une composition parfumée destinée obtenues avec l'Exemple 10.
La figure 20 est une image représentative par microscopie optique de microcapsules de silice contenant une composition parfumée obtenues avec l'Exemple 10 sans l'utilisation de surfactant et en conditions de chimie douce.
La figure 21 est un thermogramme représentatif de microcapsules de silice contenant une composition parfumée obtenues avec l'Exemple 10 et le parfum de référence.
La figure 22 est un diagramme représentatif de la distribution granulométrique par volume obtenu par diffraction laser d'un échantillon de suspension de microcapsules de silice contenant de l'HE Orange Douce obtenues avec l'Exemple 11.
La figure 23 est une image représentative par microscopie optique de microcapsules de silice contenant l'HE Orange Douce obtenues avec l'Exemple 11 sans l'utilisation de surfactant et en conditions de chimie douce.
La figure 24 est un thermogramme représentatif de microcapsules de silice contenant de l'HE Orange Douce obtenues avec l'Exemple 11.
La figure 25 est une image représentative par microscopie optique de microcapsules de silice contenant une composition parfumée obtenues avec l'Exemple 12 sans l'utilisation de surfactant et en conditions de chimie douce.

Les dessins sont donnés à titre d'exemples et ne sont pas limitatifs de l'invention. Ils constituent des représentations schématiques de principe destinées à faciliter la compréhension de l'invention et ne sont pas nécessairement à l'échelle des applications pratiques.

### DESCRIPTION DÉTAILLÉE

Avant d'entamer une revue détaillée de modes de réalisation de l'invention, sont énoncées ci-après des caractéristiques optionnelles qui peuvent éventuellement être utilisées en association ou alternativement :
A titre d'exemple, la phase huileuse est mise en émulsion avec de l'eau avant son ajout à l'issu de l'étape a) ;
A titre d'exemple, la phase huileuse est mélangée au milieu catalytique acide avant son mélange avec l'au moins un précurseur de silice, puis avec de l'eau pour former une émulsion ;
A titre d'exemple, la phase huileuse est mélangée au milieu catalytique acide et à l'au moins un précurseur de silice avant d'être mélangée à de l'eau pour former une émulsion ;
A titre d'exemple, la phase huileuse comprend au moins un actif lipophile volatil ;
A titre d'exemple, la phase huileuse comprend au moins un actif lipophile non volatil ;
A titre d'exemple, la phase huileuse comprend un solvant huileux ou dans un mélange de solvants huileux par exemple choisi parmi des solvants apolaires comme les corps gras, les huiles végétales, les triglycérides, les huiles capriques, les huiles capryliques, le myristate d'isopropyle et plus préférentiellement parmi l'un au moins parmi l'huile de ricin vierge, le miglyol ^{®} et l'huile de coco ;
A titre d'exemple, le ratio molaire polymères cationiques basiques / monomères de silice issus du précurseur de silice est supérieur ou égal à 0,3 et inférieur ou égal à 1 ; Cet intervalle permet de jouer sur la vitesse de condensation. De plus, il a été constaté de manière surprenante que l'utilisation de moins de précurseur silice et plus de polymères cationiques basiques donne des meilleures capsules ;
A titre d'exemple, le procédé comprend une étape c'), postérieure ou simultanée à l'étape c) de condensation, comprenant une étape de fonctionnalisation des capsules ;
A titre d'exemple, l'étape de fonctionnalisation des capsules comprend l'ajout d'au moins un précurseur de silice ;
A titre d'exemple, le précurseur de silice pour la fonctionnalisation représente maximum 10%, préférentiellement 7% en poids du poids total du précurseur de silice utilisé à l'étape a), préférentiellement la fonctionnalisation représente un poids inférieur ou égale à 5% du poids total du précurseur de silice utilisé à l'étape a) ;
A titre d'exemple, l'agent de condensation est ajouté à l'étape b) pour être en quantité inférieure ou égale à 100g/L dans la phase matrice, préférentiellement inférieure ou égale à 50g/L, préférentiellement inférieure ou égale à 20g/L ; les étapes a) à c) sont réalisées à une température compatible avec l'actif ou le mélange d'actifs utilisés.
A titre d'exemple, le précurseur de silice ou un mélange de précurseur de l'étape a) est choisi parmi l'un au moins parmi orthosilicate de tétraéthyle (TEOS), orthosilicate de tétraméthyle (TMOS), (3-Aminopropyl)triéthoxysilane (APTES), metasilicate de sodium, calcium silicate, triméthoxyméthylsilane (MTMOS), triéthoxyméthylsilane (MTEOS), triéthoxysilane, triméthoxysilane, triéthoxy(éthyl)silane (ETEOS), triméthoxy(éthyl)silane (ETEOS), isobutyl(triméthoxy)silane, propyl(triméthoxy)silane, orthosilicate de sodium ;
A titre d'exemple, le précurseur de silice de l'étape a) est choisi parmi l'un au moins parmi un extrait de silice biogénique, du sodium silicate ou une source naturelle d'acide ortho silicique ; A titre d'exemple, le au moins un précurseur de silice est ajouté à l'étape a) en quantité inférieure ou égale à 6000mM, préférentiellement le au moins un précurseur de silice est présent à l'étape c) en quantité inférieure ou égale à 1000mM ;
A titre d'exemple, l'anion monovalent, divalent ou trivalent est ajouté en concentration inférieure ou égale à 300mM ;
A titre d'exemple, le mélange de l'étape b) est ajouté aux nucléis obtenus à l'étape a) ;
A titre d'exemple, le procédé comprend après l'étape c) une étape d) de séparation des capsules par centrifugation ou filtration ou décantation et éventuellement une étape e) de lavage des capsules isolées et éventuellement une étape f) de séchage des capsules.

On entend par silice, du dioxyde de silicium ou des dérivés de la silice par exemple de type polysiloxane. Un siloxane étant défini comme des hydrures saturés de silicium-oxygène avec des chaînes non ramifiées ou ramifiées d'atomes alternés de silicium et d'oxygène (chaque atome de silicium est séparé de ses voisins de silicium les plus proches par des atomes d'oxygène uniques). On entend par silice, des dérivés de silice hybride tels que des matériaux inorganiques hydrides tel que par exemple organo-minéral ou des matériaux inorganiques.

On entend par « micronique » une taille de capsule comprise entre 1µm et 1000µm plus précisément entre 1µm et 450µm, dans la suite de la description, les termes micronique, micrométrique ou microcapsule s'entendent de manière identique.

Selon une possibilité, la silice produite par le présent procédé est sous forme de capsules de taille micronique. On entend par micronique que les capsules ont leur plus grande dimension inférieure ou égale à 1000 µm, plus précisément comprise entre 1 µm à 1000 µm, plus préférentiellement entre 1µm et 450µm. Préférentiellement, les capsules microniques obtenues par le procédé selon l'invention présentent une taille supérieure ou égale à 1µm et inférieure ou égale à 100µm et plus préférentiellement supérieure ou égale à 5µm et inférieure ou égale à 50µm.

Les capsules sont à base de matériau dit amorphe. On entend par amorphe que la forme du matériau est non cristalline.

Les capsules sont avantageusement de morphologie sphérique ce qui représente un avantage pour leur innocuité.

La présente invention concerne un procédé de synthèse de capsules de silice microniques dans des conditions de chimie douce.

Préférentiellement, le procédé met en œuvre une synthèse bio-inspirée.

Les capsules obtenues sont avantageusement biocompatibles.

Les capsules utilisées selon l'invention, au vu du choix de monomères, précurseurs et polymères spécifiques, ont la faculté d'être biodégradables,

Les capsules de silice obtenues suivant les modes de réalisation de l'invention ont, au vu du choix des précurseurs, la capacité à être éliminées par la dissolution en acide orthosilicique.

Les capsules obtenues par le procédé selon l'invention sont particulièrement adaptées aux applications cosmétiques, pharmaceutiques humaines ou vétérinaires, phytosanitaires, agroalimentaires, soin du linge/de la maison, les produits d'hygiène et de santé, en parfumerie, les biocides, les biostimulants et les répulsifs à usage humain ou vétérinaire.

Les capsules de silice obtenues suivant un des modes de réalisation présentent une charge de surface positive. Ceci représente un avantage notamment en termes de ciblage passif de surfaces, cellules et/ou tissus/épithélium qui présentent une charge résiduelle de surface négative. La charge de surface positive est notamment avantageuse pour la déposition sur des fibres capillaires pour des applications cosmétiques ou sur des fibres textiles pour des applications de détergence et soin du linge.

Selon l'invention, le procédé permet de produire des capsules présentant une morphologie capsulaire. On entend par capsulaire que la microcapsule comprend au moins une vésicule creuse. Selon une possibilité, la microcapsule est une vésicule creuse ou selon une autre possibilité, la microcapsule est une vésicule creuse contenant elle-même des vésicules creuses. C'est-à-dire que la capsule comprend un cœur entouré d'une coque solide, également dénommée écorce ou enveloppe.

Selon l'invention, le procédé est avantageusement configuré pour encapsuler une phase huileuse dans les microcapsules.

La phase huileuse comprenant au moins un actif.

On entend par phase huileuse, la phase contenant un actif lipophile. La phase huileuse peut être constituée de l'actif lipophile lui-même ou d'un mélange d'actifs lipophiles ou bien la phase huileuse peut comprendre l'actif lipophile dans un solvant huileux ou dans un mélange de solvants huileux ou encore un mélange d'actifs lipophiles dans un solvant huileux ou dans un mélange de solvants huileux. La phase huileuse peut comprendre un ou des additif(s).

Dans la suite de la description, le terme de phase huileuse est utilisé de manière générique pour englober ces différentes possibilités.

La phase huileuse présente un caractère lipophile. Il est connu de définir le caractère lipophile par un coefficient de partage LogP (coefficient de partage octanol/eau) supérieur ou égal à 3, préférentiellement à 4, plus préférentiellement à 5. Avantageusement, la phase huileuse présente un coefficient de partage LogP supérieur ou égal à 3, préférentiellement à 4, plus préférentiellement à 5.

L'actif est également dénommé substance active.

Selon l'invention, l'actif est lipophile.

On entend par actif lipophile, une substance active ayant une affinité pour un solvant apolaire et qui ne se mélange pas avec un solvant polaire comme l'eau. Parmi les solvants apolaires, dénommé ci-dessus solvant huileux, on peut citer les corps gras, les huiles végétales, les triglycérides, les huiles capriques, les huiles capryliques, le myristate d'isopropyle. En particulier, le solvant apolaire est choisi parmi l'un au moins parmi l'huile de ricin vierge (Inci : Ricinus communis seed oil | Cas : 8001-79-4 | EC : 232-393-8), le miglyol ^{®} (Inci : caprilic/capric triglycéride | Cas : 73398-61-5 / 65381-09-1 | EC : 277-452-2 / 265-724-3) et l'huile de coco. Cette propriété permet par exemple d'assurer que l'actif reste dans la phase huileuse. Avantageusement, l'actif présente un coefficient de partage logP supérieur ou égal à 3, préférentiellement à 4, plus préférentiellement à 5.

Selon un mode de réalisation dans lequel la phase huileuse comprend plusieurs actifs, c'est-à-dire un mélange d'actifs, il est préféré qu'au moins 50%, préférentiellement 60% et encore plus préférentiellement 80% des actifs présentent un coefficient de partage logP supérieur ou égal à 3, préférentiellement à 4, plus préférentiellement à 5.

L'actif peut être de nature diverse selon l'application. L'actif comprend au moins une molécule active ou un mélange de molécules actives. L'actif est choisi par exemple parmi un actif parfumant ou une composition parfumante, ou un extrait de plantes ou un actif cosmétique naturel ou synthétique, tel que par exemple un filtre solaire, un actif phytosanitaire, une huile essentielle ou une, ou un mélange de, phéromone(s).

Selon une possibilité, l'actif comprend au moins une substance parfumante. L'actif est un parfum ou une composition parfumante.

Selon une possibilité, l'actif comprend au moins une ou est constitué d'une huile essentielle. L'actif lipophile est soluble dans un corps gras ou une huile ou un solvant apolaire. Cette propriété permet d'assurer que l'actif est dispersé dans la phase huileuse.

L'actif lipophile peut être volatil. On entend par actif lipophile volatil, une substance capable de se vaporiser d'un état liquide à l'état gazeux. Il est connu d'utiliser la pression de vapeur et la température d'ébullition comme indicateurs de la volatilité d'une substance. Par exemple, l'actif présente une pression de vapeur supérieure à 0.01 kPa à une température de 20 °C ou une température d'ébullition inférieure à 250°C à la pression standard de 101,3 kPa. Parmi les actifs lipophiles volatiles on peut citer par exemple les huiles essentielles (par exemple d'orange douce), les dérivés terpéniques (par exemple le limonène), le tetrahydromyrcénol.

L'actif lipophile peut être non volatil. On entend actif lipophile non volatil une substance difficilement vaporisable d'un état liquide à l'état gazeux. Par exemple, l'actif présente une pression de vapeur inférieure ou égale à 0.01 kPa à une température de 20 °C ou une température d'ébullition supérieure ou égale à 250°C, à la pression standard de 101,3 kPa. Parmi les actifs lipophiles non volatiles on peut citer par exemple le cédryl acétate.

On entend par rendement d'encapsulation, le ratio de la phase huileuse encapsulée sur la masse de phase huileuse à encapsuler. Il est exprimé en pourcentage.

On entend par taux d'emport, le pourcentage massique de phase huileuse contenue dans les capsules.

On entend par rendement global le rapport de la masse des capsules obtenues sur la masse théorique de capsules attendues. La masse théorique de capsules attendues est la somme de la masse de phase huileuse à encapsuler et de la quantité attendue de matériau d'encapsulation, le matériau d'encapsulation étant issu du précurseur de silice.

Le procédé comprend avantageusement 3 étapes.

Préférentiellement, les trois étapes : étape a), étape b) et étape c) sont réalisées en milieu aqueux.

Selon un mode de réalisation, le procédé comprend une étape a) de préparation des nucléis de silice. Selon l'invention, l'étape a) est une hydrolyse d'au moins un précurseur de silice. L'étape a) est réalisée dans un milieu aqueux et avantageusement en milieu aqueux acido-catalysé.

Selon un mode de réalisation, le au moins un précurseur de silice est choisi parmi des précurseurs :
d) de type siloxy répondant à la formule (R)xSi(O-R1)₄₋ₓ avec R1= groupement alkyle (C1-C4) ou hydroxyle, et R= groupement type alkoxy, hydrogène, alkyle linéaire ou branché ou un alcène, pouvant présenter un groupement fonctionnel type amine, carboxyle, thiol, hydroxyle, époxy, et/ou
e) de type orthosilicate inorganique répondant à la formule SiO₄M ou (M2O)ₓ·(SiO2)_{y} avec x=1 ou 2, y = 1 ou 2 avec M=métal tel que Ca, Na par exemple, Ca₂SiO₄ ou 2CaOSiO₂, ou Na₄SiO₄, et/ou
f) de type orthosilicate organique comme (CH₃)₄SiO₄ et (CH₂CH₃)₄SiO₄.

Préférentiellement, le au moins un précurseur de silice est avantageusement choisi parmi l'un au moins parmi orthosilicate de tétraéthyle (TEOS), orthosilicate de tétraméthyle (TMOS), (3-Aminopropyl)triéthoxysilane (APTES), métasilicate de sodium, calcium silicate, triméthoxyméthylsilane (MTMOS), triéthoxyméthylsilane (MTEOS), triéthoxysilane, triméthoxysilane, triéthoxy(éthyl)silane (ETEOS), triméthoxy(éthyl)silane (ETMOS), isobutyl(triméthoxy)silane, propyl(triméthoxy)silane, orthosilicate de sodium.

Selon une possibilité, le précurseur de silice est un extrait de silice biogénique tel que par exemple issue des résidus de riz ou de diatomées, du sodium silicate ou une source naturelle d'acide ortho silicique.

Préférentiellement, la quantité de précurseur de silice ajoutée lors de l'étape a) est inférieure ou égale à 6000 mM, préférentiellement inférieure ou égale 5000mM, plus préférentiellement inférieure ou égal à 4500mM.

Préférentiellement, la quantité de précurseur de silice lors de l'étape c) est inférieure ou égale à 1000mM, plus préférentiellement inférieure à 800mM, préférentiellement inférieur ou égal à 450mM.

Selon un mode de réalisation, la quantité d'acide ajouté est avantageusement inférieure ou égale à 0,05 équivalent acide. Plus précisément, la quantité d'acide est inférieure ou égale à 0,02, plus préférentiellement supérieure ou égale à 0,001 et inférieure ou égale à 0,015 équivalent acide. Les quantités d'acide sont données en équivalent acide par rapport au précurseur de silice. Selon une possibilité, l'acide est choisi parmi un acide carboxylique faible ou un acide fort. À titre préféré, l'acide est choisi parmi l'acide formique, l'acide acétique ou l'acide chlorhydrique. Avantageusement, le pH du milieu aqueux dans lequel les nucléis sont préparés est à pH inférieur ou égal à 5, préférentiellement inférieur ou égal à 4, préférentiellement inférieur ou égal à 3. Avantageusement, le pH est inférieur ou égal à 3 avec une concentration d'acide minimale de sorte à ce placer en milieu acido-catalysé permettant d'assurer des conditions de chimie douce au procédé.

À titre d'exemple, les nucléis sont formés au maximum de quelques dizaines d'atomes, par exemple, un nucléi de silice présente une taille de l'ordre de quelques nanomètres de diamètre et préférentiellement inférieur à 20 nm.

Le procédé selon l'invention est avantageusement configuré pour encapsuler l'actif dans une micro-capsule, l'actif, de manière plus générale la phase huileuse, est dans le cœur de la capsule.

A cet effet, le procédé selon l'invention comprend l'ajout d'une phase huileuse comprenant, ou constituée, d'au moins un actif lipophile.

L'ajout de la phase huileuse est réalisé soit au cours de l'étape a), soit à l'issue de l'étape a) et avant l'étape c) décrite ci-après.

Selon une première possibilité, la phase huileuse est ajoutée aux nucléis obtenus à l'étape a). La phase huileuse est avantageusement ajoutée aux nucléis obtenus à l'étape a) avant le mélange à la phase matrice obtenue à l'étape b). Avantageusement, la phase huileuse est ajoutée à la fin de l'étape a), lorsque les nucléis sont formés de sorte à limiter les perturbations sur la formation des nucléis lors de l'étape a). Ce procédé conduit avantageusement à l'obtention de capsules de morphologie capsulaire. Ce procédé permet notamment d'assurer un temps de contact limité entre l'actif et le milieu acide ce qui est très utile dans le cas où l'actif est sensible aux acides. Selon une deuxième possibilité, la phase huileuse est ajoutée aux nucléis obtenus à l'étape a). Le précurseur de silice est mélangé au milieu catalytique acide. Des nucléis commencent à se former. La phase huileuse est avantageusement ajoutée à l'eau. Une émulsion de la phase huileuse dans la phase aqueuse ne contenant pas le précurseur de silice, ni l'acide, est formée par agitation. Le précurseur de silice en milieu catalytique acide ayant formé des nucléis est ensuite ajouté à l'émulsion, préférentiellement sous agitation. Le précurseur de silice en milieu catalytique acide a formé des nucléis qui lors du mélange avec l'émulsion viennent s'agencer à l'interface phase huileuse/phase aqueuse permettant de stabiliser l'émulsion.

Selon une troisième possibilité, la phase huileuse est ajoutée à l'étape a) de préparation des nucléis. La phase huileuse est avantageusement ajoutée au milieu catalytique acide aqueux. Le mélange de la phase huileuse avec le milieu catalytique acide aqueux et ne contenant pas le précurseur de silice est formée par agitation. Le précurseur de silice est ensuite ajouté au mélange. Des nucléis commencent à se former. Puis l'ensemble est ajouté à l'eau, avantageusement sous agitation, pour former une émulsion.

Selon une quatrième possibilité, la phase huileuse est ajoutée à l'étape a) de préparation des nucléis. La phase huileuse est avantageusement ajoutée au précurseur de silice en milieu catalytique acide aqueux. Les nucléis commencent à se former. Le mélange nucléis, phase huileuse est ajouté dans l'eau sous agitation pour former une émulsion de la phase huileuse dans la phase aqueuse contenant les nucléis, avantageusement par agitation.

Le mélange de la phase huileuse dans une phase aqueuse se fait sous agitation. À titre d'exemple, le mélange phase huileuse-phase aqueuse, la phase aqueuse étant préférentiellement de l'eau, est agité pour un minimum de 30 secondes et jusqu'à 5 minutes. À titre d'exemple, le mélange est agité à une vitesse minimale de 2000 rpm et jusqu'à 10000 rpm. À titre d'exemple, le mélange présente une concentration massique d'actif(s) dans l'eau de l'ordre de 10% par poids.

Selon l'invention, la phase huileuse est ajoutée dans un ratio en poids phase huileuse / précurseur de silice minimal de 1/1 et maximal de 7/1, par exemple de 4.2/1.

De manière surprenante ce ratio permet d'assurer un taux d'emport de la phase huileuse supérieur à 85%.

Le ratio en poids phase huileuse / précurseur de silice est calculé en prenant en considération la quantité, en poids, de précurseur de silice hydrolysé. En effet, c'est le précurseur de silice hydrolysé qui va réagir et qui est donc pris en considération dans le ratio.

Cette quantité de précurseur de silice sous forme hydrolysé est égale à :
(Quantité de précurseur de silice / Masse moléculaire du précurseur de silice) x Masse moléculaire du précurseur de silice hydrolysé.

Pour le calcul du ratio, la phase huileuse s'entend de l'actif ou des actifs et du ou des solvants huileux.

Avantageusement, le procédé ne comprend pas d'ajout d'un tensio-actif, également dénommé surfactant en anglais. Le procédé selon l'invention n'a pas besoin de tensio-actif pour stabiliser les gouttelettes de la phase huileuse lors de l'émulsification avant formation de la capsule. Selon un mode de réalisation, le procédé comprend une étape b) de formation d'une phase matrice. La phase matrice s'entend comme la phase destinée à permettre la formation de silice. La matrice est formée par mélange d'au moins un agent de condensation et d'au moins un anion monovalent, divalent, ou trivalent ou un mélange d'anions monovalent, divalent, ou trivalent. L'agent de condensation est avantageusement un polymère polycationique basique branché ou linéaire. Ceci représente un avantage par rapport à l'utilisation plus classique de base forte type ammoniaque. Le choix d'un polymère polycationique basique permet de s'affranchir du besoin d'ajuster le pH par ajout d'une base forte type ammoniaque pour initier la condensation. Cela permet d'avoir un procédé plus écologique de type chimie douce. Le procédé ne comprend pas d'étape uniquement destinée à l'ajustement du pH.

Préférentiellement, le polymère est choisi pour avoir un poids inférieur ou égal à 800 KDa. Selon un aspect, ledit polymère comprend les polyaminoacides notamment basiques comme les polyarginines, les polylysines et polyhistidines, mais également les polyallylamines, les polyéthylènes imines (PEI), les polypropylènes imines, les dérivés de polysaccharides de type galactomannane, fructo-oligosaccharides, d'oligofructoses ou des mélanges de ceux-ci. L'agent de condensation peut découler de la modification chimique d'un des polymères cités ci-dessus et ceci afin de moduler ses propriétés physico-chimiques.

Selon un aspect, l'agent de condensation est plus précisément un polymère riche en fonction amines primaires, secondaires ou tertiaires c'est à dire comprenant un nombre de résidus amines supérieur ou égal à 3.

Dans le cas des polyéthylènes imines (PEI) branchés ou linéaires, ils sont constitués du motif répétitif éthylène imine type (C2H5N)n de masse molaire 43.04g/mol. À titre d'exemple, un polymère peut être le diéthylènetriamine ou tous ses homologues supérieurs.

L'agent de condensation peut aussi être un PEI branché de formule suivante : H(NHCH₂CH₂)ₙNH₂)n de poids moléculaire compris entre 10000 et 750000, notamment entre 25000 et 750000 ou un mélange de PEI tels que par exemple des mélanges de PEI à 10kDa et 25kDa.

Selon un aspect, l'agent de condensation est un dendrimère polyaminé de génération supérieure à 1 contenant des motifs type [-CH₂CH₂N(CH₂CH₂CH₂NH₂)₂]₂, à titre d'exemple le DAB-Am-4, Polypropylenimine tetramine dendrimère, génération 1.

Dans le cas des dérivés de polysaccharides à galactomannane, la gomme de guar modifiée ou non est préférée. La gomme de guar peut être modifiée par un groupement basique amine de synthèse ou non, à titre d'exemple un groupement ammonium quaternaire.

Dans le cas des dérivés d'oligofructoses, l'inuline est préférée. L'inuline peut être modifiée par un groupement basique amine de synthèse ou non, à titre d'exemple un groupement ammonium quaternaire.

Selon une possibilité, l'agent de condensation est un mélange de PEI et de guar et/ou d'inuline. Préférentiellement, l'agent de condensation est ajouté lors de l'étape b) pour atteindre une concentration massique finale à l'étape b) inférieure ou égale à 100g/L, plus précisément inférieure ou égale à 50g/L, préférentiellement inférieure ou égale à 20g/L. Le procédé selon l'invention permet d'utiliser une faible quantité d'agent de condensation et donc obtenir un pH de condensation basique le plus proche des conditions physiologiques, préférentiellement de l'ordre d'un pH de 10, préférentiellement de l'ordre de 9, plus préférentiellement de l'ordre de 8. L'agent de condensation est avantageusement sélectionné pour présenter des groupements chimiques favorables à la formation d'interactions non covalentes telles que par exemple des liaisons électrostatiques et/ou type liaisons hydrogènes. De cette manière, l'agent de condensation assiste de manière contrôlée la polymérisation des monomères de silice.

L'agent de condensation est avantageusement un polymère bio-inspiré ou naturel ou bio-sourcé. L'agent de condensation est avantageusement recyclé à la fin du procédé selon l'invention. Le recyclage de l'agent de condensation est réalisé par des procédés connus de l'homme de l'art tels que des techniques de filtration sur gel d'exclusion et/ou résines échangeuses d'ions, ultrafiltration sur des membranes de poids moléculaire nominal limite adaptées. Le recyclage de l'agent de condensation représente un avantage indéniable.

L'agent de condensation tel que notamment le PEI ou la gomme de guar modifiée ou non, agit à la fois comme un catalyseur accélérant la réaction de condensation et une matrice qui contrôle la réaction et la formation des capsules pour le grossissement des nucléis à partir de points de nucléation apportés par l'association polymère-anion tel que le phosphate.

Selon l'invention, les quantités de polymères cationiques basiques et de monomères de silice issus du précurseur de silice sont choisies pour être en faveur des monomères de silice. Le ratio molaire polymères cationiques basiques / monomères de silice issus du précurseur de silice est supérieur ou égal à 0,3 et inférieur ou égal à 1 ; Ce ratio se calcule avec les quantités molaires de polymères cationiques basiques et les quantités molaires de monomères de silice. Ce sont les quantités molaires qui sont mises en jeu lors de réaction chimique. Le monomère de silice correspond au précurseur de silice hydrolysé. Le calcul se base sur le poids moléculaire après la perte des fonctions hydrolysables du précurseurs de silices (fonctions éthoxy -OC2H5) par exemple pour le MTEOS hydrolysé 94.1410 g/mol et pour le TEOS hydrolysé 96.1130 g/mol Selon un mode de réalisation, le au moins un anion monovalent, divalent, trivalent est un sel anionique. À titre préféré, l'anion monovalent, divalent, trivalent est choisi parmi l'un au moins parmi un sel de phosphate, un sel de citrate, ou un sel de tartrate, un sel de sulfate, ou un sel de nitrate.

À titre d'exemple, le sel de phosphate est choisi parmi phosphate de sodium, phosphate de magnésium, phosphate de potassium, phosphate de calcium.

À titre d'exemple, le sel de citrate est choisi parmi le citrate de sodium, citrate de potassium, citrate de calcium, citrate de magnésium.

À titre d'exemple, le sel de tartrate est choisi parmi le tartrate de sodium, tartrate de potassium, tartrate de calcium, tartrate de sodium et potassium, tartrate de choline, tartrate d'ammonium.

Préférentiellement, l'anion monovalent, bivalent, ou trivalent est ajouté lors de l'étape b) pour atteindre une concentration finale inférieure ou égale à 300mM, plus précisément 200mM. L'ajout d'au moins un anion ou d'un mélange d'anions selon cette sélection de concentration permet d'obtenir l'effet de l'anion tout en limitant l'impact sur le pH du milieu de condensation.

Le au moins un anion, ou mélange d'anions, monovalent, divalent ou trivalent est ajouté à l'agent de condensation pour notamment assurer des interactions électrostatiques avec l'agent de condensation et permettre la formation de la matrice, notamment pour contrôler la formation de capsules.

Selon un mode de réalisation, le procédé comprend une étape c) de condensation en milieu basique. L'étape de condensation assure la condensation des nucléis obtenus à l'étape a) de manière contrôlée grâce à la matrice obtenue à l'étape b). L'étape de condensation permet aux nucléis obtenus à l'étape a) de former un réseau solide pour obtenir des capsules.

L'étape c) comprend avantageusement le mélange des nucléis de silice et phase huileuse avec la matrice, préférentiellement sous agitation.

Selon une possibilité, les nucléis obtenus à l'étape a) additionnés de la phase huileuse, l'ensemble étant dénommé phase hydrolyse, sont ajoutés à la matrice obtenue à l'étape b), également dénommée phase matrice.

Selon une autre possibilité, la phase matrice obtenue à l'étape b) est ajoutée aux nucléis obtenus à l'étape a) additionnés de la phase huileuse.

Selon un mode de réalisation, la phase hydrolyse est ajoutée en une seule portion à la phase matrice. Selon une possibilité, l'ajout se fait avec un débit contrôlé ou sans contrôle directement en une fois.

Avantageusement, l'étape c) de condensation est réalisée à pH basique, dit modéré, c'est-à-dire inférieur ou égal à 10. Préférentiellement, à pH inférieur ou égal à 9 encore plus préférentiellement inférieur ou égal à 8 et supérieur à 7.

Selon un mode de réalisation avantageux de la présente invention, le procédé de synthèse, plus préférentiellement les étapes a), b) et c) et éventuellement l'étape de fonctionnalisation décrite ci-après, est réalisé sans chauffage ou refroidissement. Préférentiellement, le procédé est configuré pour maintenir une température compatible avec l'actif ou le mélange d'actifs utilisés.

Selon une possibilité, l'ajout du précurseur de silice peut être réalisé en une ou plusieurs fois, avec ou sans refroidissement.

Selon un mode de réalisation, le procédé selon l'invention assurant la synthèse de capsules microniques de silice comprend avantageusement une étape c'), postérieure ou simultanée à l'étape c) de condensation, comprenant une étape de fonctionnalisation des capsules. Dans le cas où, l'étape c') est postérieure à l'étape c), elle est avantageusement directement postérieure, c'est à dire que l'étape c) et l'étape c') sont successives, préférentiellement sans étape intermédiaire. L'étape de fonctionnalisation permet de modifier la surface des capsules de silice pour aboutir à une fonctionnalisation de surface ciblée et attribuer de nouvelles propriétés aux capsules. L'étape de fonctionnalisation comprenant avantageusement l'ajout d'un précurseur de silice. Selon un exemple, le précurseur de silice est choisi parmi l'un au moins parmi TEOS (orthosilicate de tétraéthyle), TMOS (orthosilicate de tétraméthyle), MTMOS (méthyltriméthoxysilane), MTEOS (méthyltriéthoxysilane), ETEOS (éthyltriéthoxysilane), ETMOS (éthyltriméthoxysilane), APTES ((3-aminopropyl)triéthoxysilane), orthosilicate de sodium ou métasilicate de sodium. Le précurseur de silice de l'étape de fonctionnalisation peut être identique ou différent du précurseur de silice utilisé à l'étape a).

Selon une possibilité, l'étape de fonctionnalisation comprenant avantageusement l'ajout d'un précurseur de silice de type organosilane comme agent de couplage. Selon un exemple, le précurseur de silice est choisi parmi l'un au moins parmi précurseur de silice présentant au moins un groupement agent de couplage choisi parmi un amino, isocyanate, mercapto, vinyl, acrylate. Par exemple le précurseur agent de couplage est l'APTES ((3-Aminopropyl)triéthoxysilane), le vinyltriméthoxysilane, vinyltriéthoxysilane, le glycidoxypropyltrimethoxysilane, le glycidoxypropyltriethoxysilane, le 3-mercaptopropyltrimethoxysilane. Le précurseur de silice de l'étape de fonctionnalisation peut être identique ou différent du précurseur de silice utilisé à l'étape a).

Selon un mode de réalisation, le procédé de l'invention assurant la synthèse de capsules microniques de silice comprend avantageusement une étape d), postérieure à l'étape c) de condensation, préférentiellement postérieure à l'étape c') si présente, comprenant une étape de séparation des capsules. L'étape de séparation permet de dissocier les microcapsules des éventuels nucléis non condensés, de phase matrice et/ou d'actifs résiduels. L'étape de séparation des capsules peut être par exemple réalisée par centrifugation, par filtration frontale, par décantation ou filtration tangentielle à courant transversal.

Selon un mode de réalisation, le procédé selon l'invention assurant la synthèse de capsules micrométriques de silice comprend une étape e), postérieure à l'étape c), préférentiellement postérieure à l'étape c') si présente, et éventuellement à l'étape d) de séparation qui permet une purification des microcapsules de silice par lavage ou extraction chimique. L'étape de purification est destinée à permettre la suppression de résidus organiques issus du procédé. Cependant, du fait du procédé de synthèse de l'invention, réalisé en conditions de chimie douce notamment bio-inspirées notamment avantageusement sans solvant organique, les résidus organiques éventuels sont non nocifs et n'impactent pas les propriétés des capsules de ce fait ils ne doivent pas obligatoirement être retirés des microcapsules et peuvent apporter de nouvelles propriétés aux capsules telles que la déposition. Par exemple, les polymères cationiques ne doivent pas obligatoirement être retirés des microcapsules pour favoriser la déposition sur des surfaces chargées. À titre d'exemple, l'étape de purification peut être effectuée par des cycles de lavage et/ou centrifugation.

Selon un mode de réalisation, le procédé selon l'invention assurant la synthèse de capsules micrométriques de silice comprend une étape de séchage des microcapsules f) postérieure à l'étape c) de condensation préférentiellement postérieure à l'étape c') si présente, et éventuellement des étapes d) de séparation et e) de purification. L'étape de séchage permet d'obtenir des microcapsules sous la forme sèche, ce qui peut être un avantage en termes de stockage par exemple. L'étape de séchage peut être réalisée par atomisation ou spray drying. Avantageusement, cette étape de séchage par atomisation ou spray-drying peut conduire à une étape de mise en forme mécanique des microcapsules de silice et d'actifs.

### Exemple 1

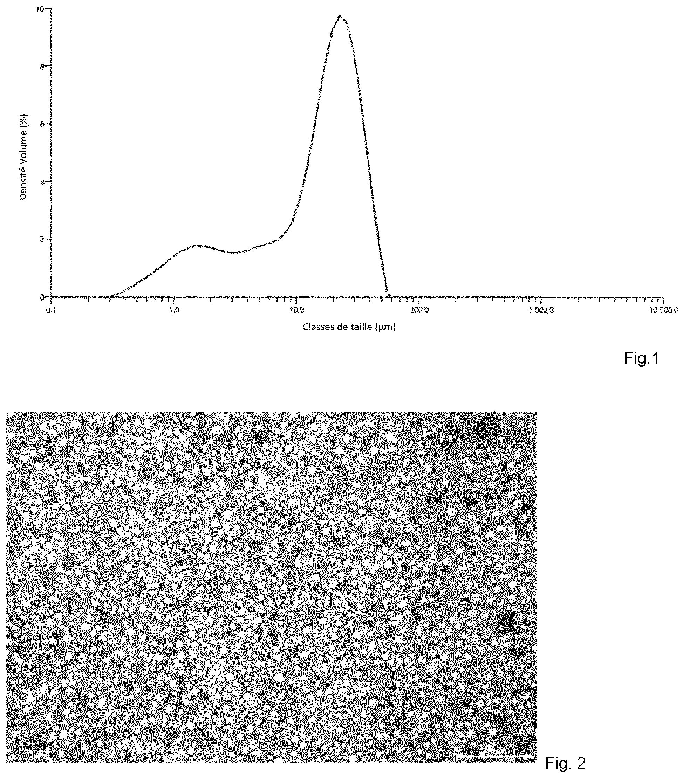

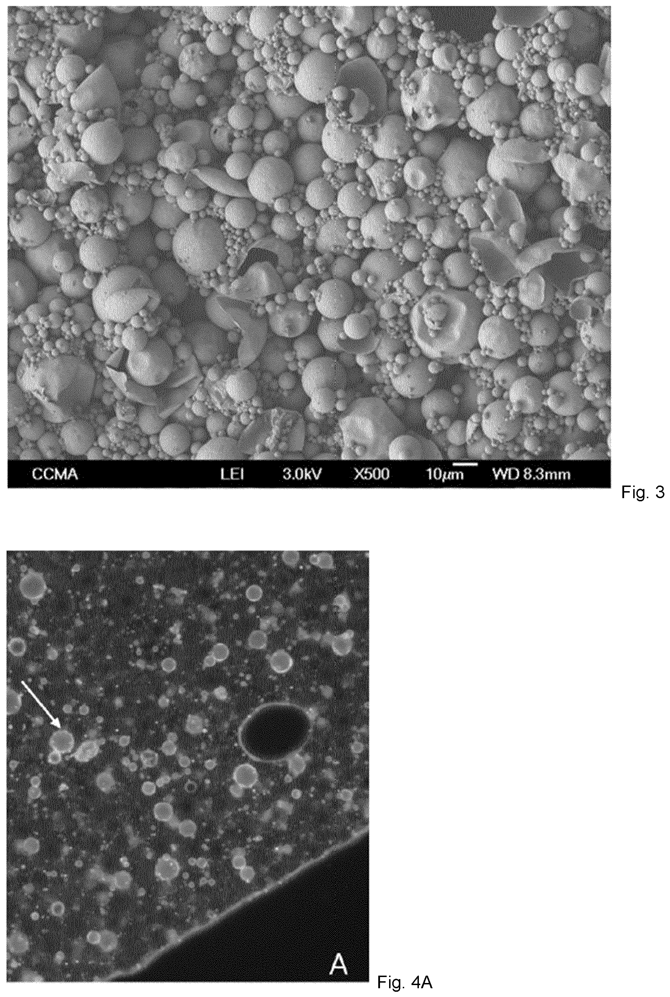

12.6 g de CBD broad spectrum 32% (huile de coco) sont émulsionnés dans 46.7 g d'eau avec un mélangeur à fort cisaillement (Turrax T25). Sous cisaillement, 5.7 g de précurseur MTEOS préalablement hydrolysé en conditions acides catalytiques (pH 1.5) sont mélangés à l'émulsion jusqu'à obtenir la taille d'émulsion souhaitée, puis l'émulsion est traitée par 30.3 g de solution aqueuse contenant 1.1 g de polyéthylène imine 25kDa et 2.0 g de sels de phosphate de sodium. Les capsules sont formées instantanément et présentent des tailles entre 1-50µm suivant la force de cisaillement et la géométrie de l'outil de dispersion utilisées. Les capsules sont séparées par centrifugation. La distribution granulométrique est mesurée par granulométrie laser (Malvern Mastersizer 3000E, Figure 1). La morphologie des particules est déterminée par microscopie optique et MEB. Les particules ont une morphologie sphérique (Figure 2) et une structure de type cœur/écorce (Figure 3). 14.8 grammes de microcapsules CBD broad spectrum sont obtenus. Le contenu des capsules en phase huileuse est de 81% par poids après extraction. Le contenu en CBD broad spectrum est 27.8% (par dosage HPLC).

La quantité de précurseur hydrolysé est de 3,01g. le ratio poids phase huileuse / précurseur de silice est de 3,01 / 12, 6g soit 4,19.

### Marquage par fluorescence

Des capsules obtenues avec l'Exemple 1 ont été traitées avec 0.25 mL d'un complexe FITC-APTES dans l'éthanol (0.085% équivalent mol au silicium) avant séparation pour marquage par fluorescence.

### Formulation en crème de jour 0.3% CBD

Des capsules obtenues avec l'Exemple 1 ont été formulées en crème de jour. La phase aqueuse A et phase huileuse B (voir tableau 1 composition) sont préparées séparément par mélange des ingrédients sous agitation magnétique (600 tr/min) et chauffage au bain marie à 70°C. La phase B est ajoutée à la phase A sous agitation par tige défloculeuse (800 tr/min). L'émulsion huile/eau est maintenue sous agitation et chauffage 10 min, puis refroidie à 30°C sous simple agitation magnétique (500 rpm).

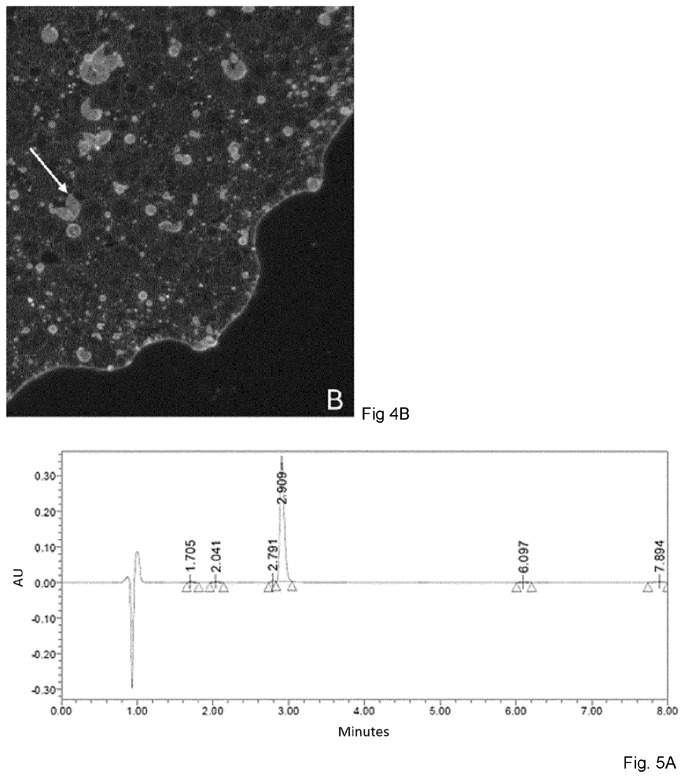

La microscopie à fluorescence obtenue à partir de microcapsules fluorescentes obtenues avec l'Exemple 1 permet d'observer la conservation de la structure des particules après formulation (Figure 4A) et la capacité de rupture à l'étalement (Figure 4B).

**[Table 1]**

| | Nom INCI | Masse (g) |
|---|---|---|
| Phase A | Aqua | 35.2 |
| | Glycerin | 1.5 |
| | Cannabidiol (*microencapsulé*) | 0.3 |
| Phase B | Glyceryl stearate | 6 |
| | Stearyl alcohol | 1 |
| | Ceteareth-20 | 1 |
| | Caprylic/capric TG | 4 |
| | Prunus dulcis oil | 1 |

### Stabilité du CBD microencapsulé et formulé

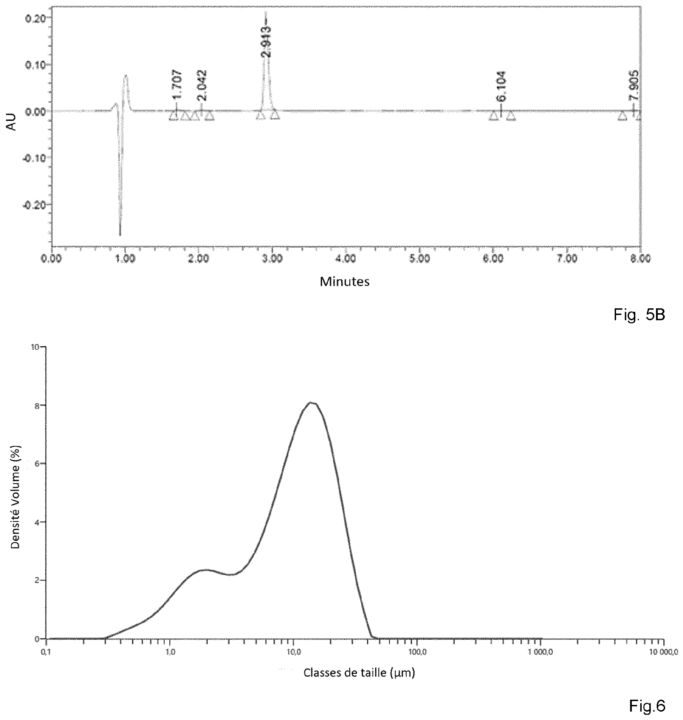

La stabilité d'échantillons de CBD microencapsulé obtenus avec l'Exemple 1 en suspension dans l'eau et dans la formulation crème de jour a été confirmée par dosage HPLC après stockage jusqu'à 8 semaines à 37°C (conditions accélérées) et jusqu'à 10j à 50C° (conditions stressées) avec l'absence de dégradation significative (<5%) du pic du CBD (TR : 2.9min) par rapport au contrôle stocké à 4°C (Figures 5A et 5B)

### Exemple 2

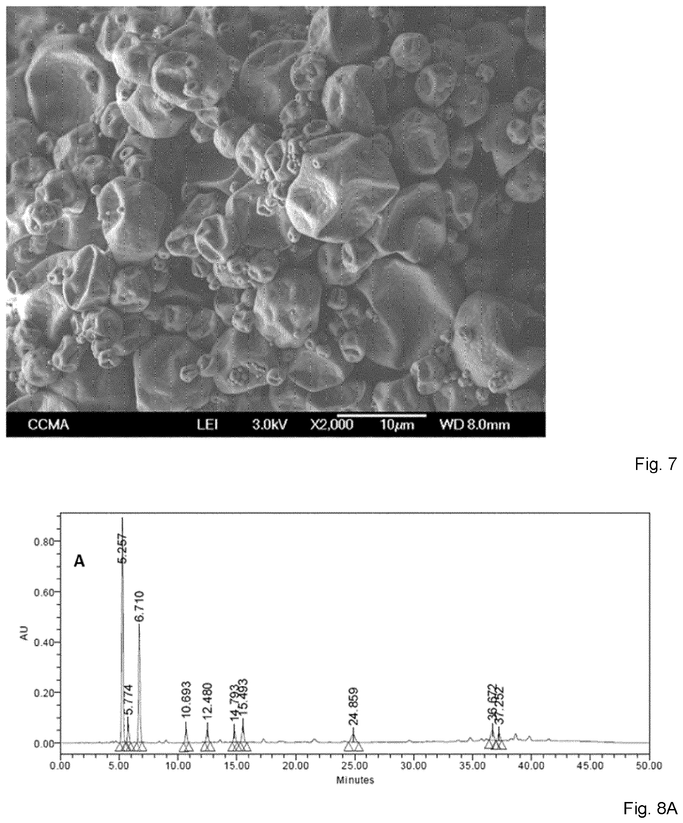

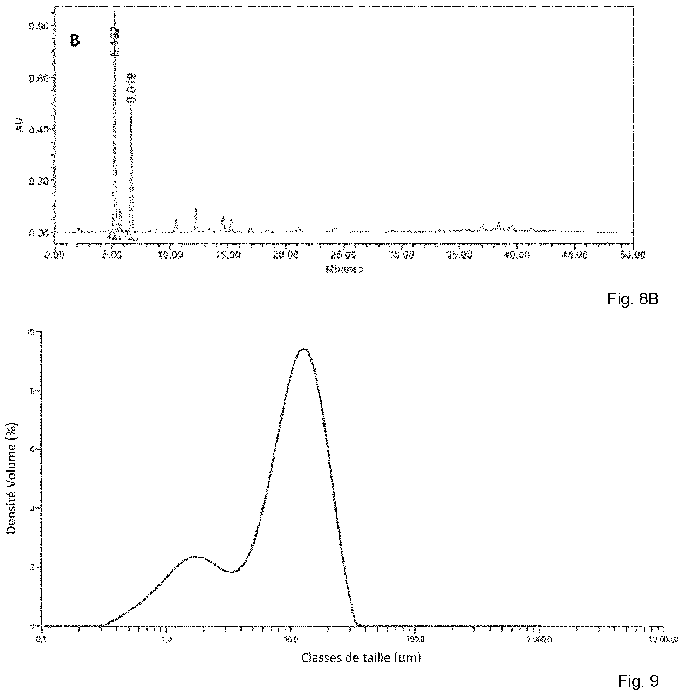

43.9 g de HE Pélargonium dans 81.5g miglyol 812 sont émulsionnés dans 513 g d'eau avec un mélangeur à fort cisaillement (Turrax T25). Sous cisaillement, 57.2 g de précurseur MTEOS préalablement hydrolysé en conditions acides catalytiques (pH 1.5) sont mélangés à l'émulsion jusqu'à obtenir la taille d'émulsion souhaitée, puis l'émulsion est traitée par 280 g de solution aqueuse contenant 11.2 g de polyéthylène imine 25kDa et 21.0 g de sels de phosphate de sodium. Les capsules sont formées instantanément et présentent des tailles entre 1-50µm suivant la force de cisaillement et la géométrie de l'outil de dispersion utilisées. La distribution granulométrique est mesurée par granulométrie laser (Malvern Mastersizer 3000E, Figure 6). La morphologie des particules est déterminée par MEB. Les particules ont une morphologie sphérique et une structure de type cœur/écorce (Figure 7). Les capsules sont séparées par centrifugation. 148.6 grammes de microcapsules HE Pélargonium sont obtenus. Le contenu des capsules en phase huileuse est de 85.5% par poids après extraction. Le contenu en HE Pélargonium est 29.9% par dosage HPLC et conforme au profil de l'HE Pélargonium (Figures 8A et 8B).

La quantité de précurseur hydrolysé est de 30,20g. La quantité de phase huileuse est 125,4g le ratio poids phase huileuse / précurseur de silice est 4,15.

### Exemple 3

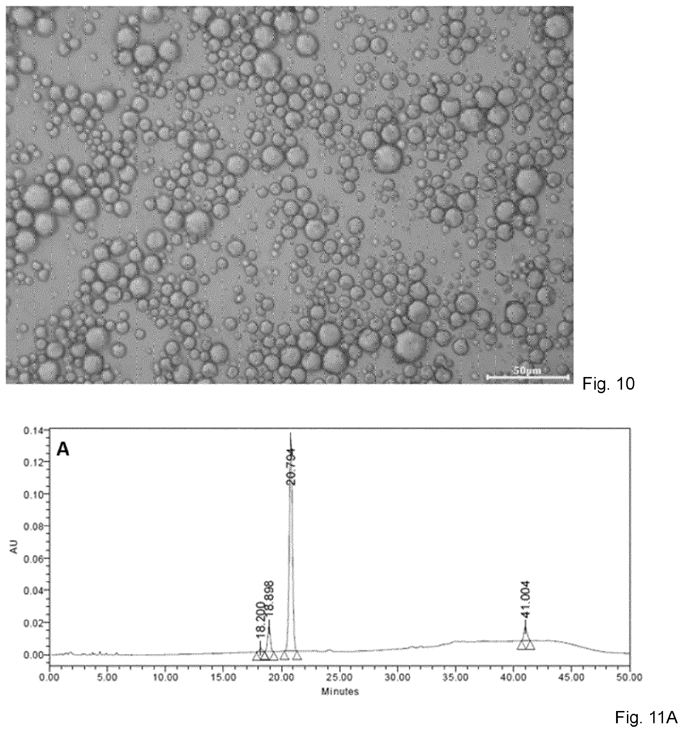

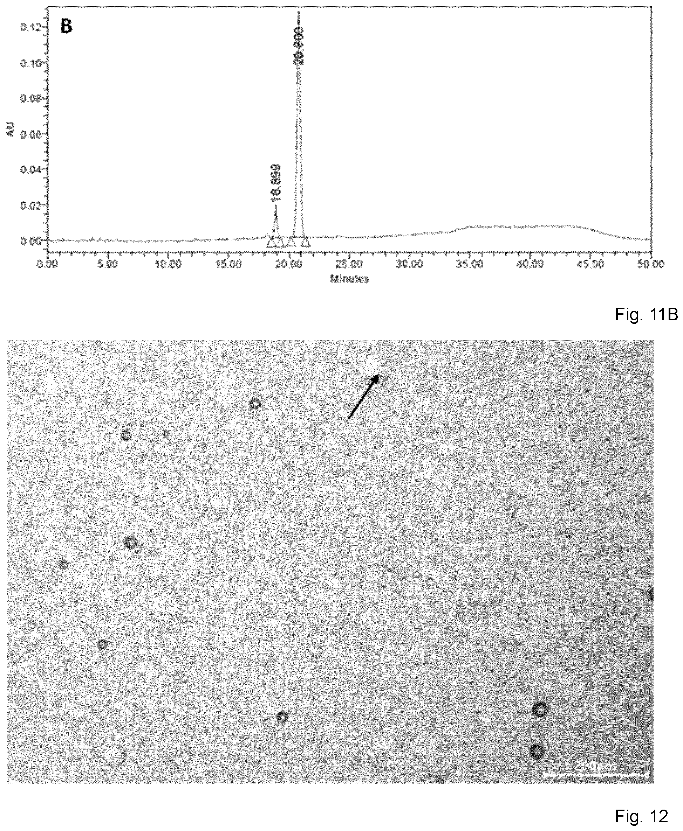

43.9 g de HE Orange douce dans 81.5 g miglyol 812 sont émulsionnés dans 513 g d'eau avec un mélangeur à fort cisaillement (Turrax T25). Sous cisaillement, 57.2 g de précurseur MTEOS préalablement hydrolysé en conditions acides catalytiques (pH 1.5) sont mélangés à l'émulsion jusqu'à obtenir la taille d'émulsion souhaitée, puis l'émulsion est traitée par 280 g de solution aqueuse contenant 11.2g de polyéthylène imine 25kDa et 21.0 g de sels de phosphate de sodium. Les capsules sont formées instantanément et présentent des tailles entre 1-50µm suivant la force de cisaillement et la géométrie de l'outil de dispersion utilisées. La distribution granulométrique est mesurée par granulométrie laser (Malvern Mastersizer 3000E, Figure 9). La morphologie des particules est déterminée par MEB. Les particules ont une morphologie sphérique et une structure de type cœur/écorce (Figure 10). Les capsules sont séparées par centrifugation. 111.0 grammes de microcapsules HE Orange douce sont obtenus. Le contenu des capsules en phase huileuse est de 89.7% par poids après extraction. Le contenu en HE Orange douce est 31.6% par dosage HPLC et conforme au profil de l'HE Orange Douce (Figures 11A et 11B).

La quantité de précurseur hydrolysé est de 30,20g. La quantité de phase huileuse est 125,4g le ratio poids phase huileuse / précurseur de silice est 4,15.

### Exemple 4

2.21 g de HE Orange douce et 4.10 g miglyol 812 sont émulsionnés dans 54.24 g d'eau avec un mélangeur à fort cisaillement (Turrax T25). Sous cisaillement, 9mL de précurseur MTEOS préalablement hydrolysé en conditions acides catalytiques (pH 1.5) sont mélangés à l'émulsion jusqu'à obtenir la taille d'émulsion souhaitée, puis l'émulsion est traitée par 15 g de solution aqueuse contenant 1.127 g de polyéthylène imine 25kDa et 13.05 g de sels de phosphate de sodium. Des capsules sont formées instantanément et observées en présence d'une quantité significative d'huile essentielle non encapsulée comme observée par microscopie optique (Figure 12).

La quantité de précurseur hydrolysé est de 4,26g. La quantité de phase huileuse est 6,31g le ratio poids phase huileuse / précurseur de silice est 1,48.

### Exemple 5

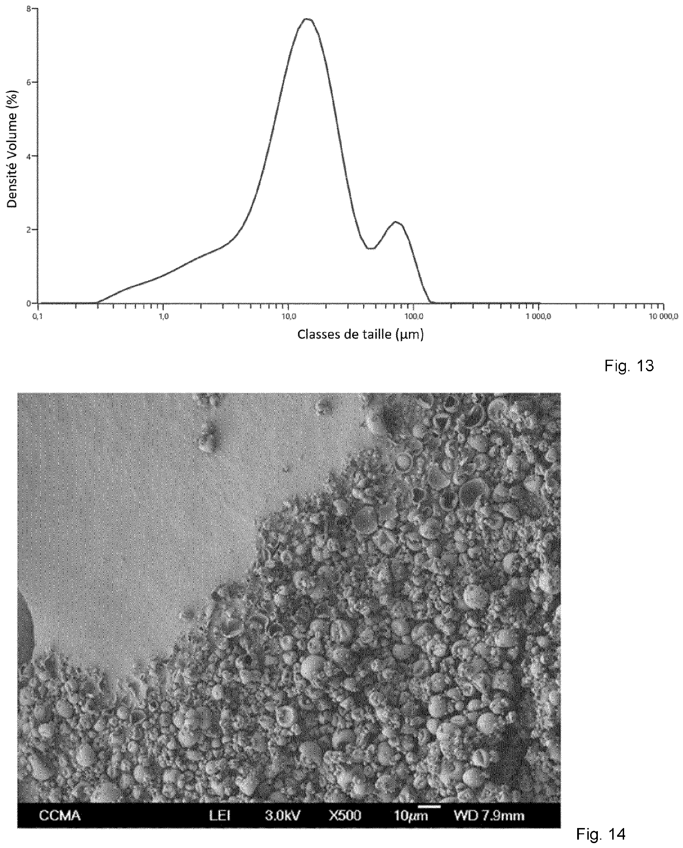

3.22 g de composition parfumée (1) dans 1.0 g miglyol 812 sont émulsionnés dans 42.3 g d'eau avec un mélangeur à fort cisaillement (Turrax T25). Sous cisaillement, 8.0 g de précurseur MTEOS préalablement hydrolysé en conditions acides catalytiques (pH1.5) sont mélangés à l'émulsion jusqu'à obtenir la taille d'émulsion souhaitée, puis l'émulsion est traitée par 42.1 g de solution aqueuse contenant 1.7 g de polyéthylène imine 25kDa et 3.1 g de sels de phosphate de sodium. Les capsules sont formées instantanément et présentent des tailles entre 1-50µm suivant la force de cisaillement et la géométrie de l'outil de dispersion utilisées. Après 5 minutes, 0.45 g de APTES sont ajoutés au mélange sous homogénéisation. La distribution granulométrique est mesurée par granulométrie laser (Malvern Mastersizer 3000E, Figure 13). La morphologie des particules est déterminée par MEB. Les particules ont une morphologie sphérique et une structure de type cœur/écorce (Figure 14). Les capsules sont séparées par centrifugation. 5.7 grammes de microcapsules sont obtenus. Le contenu des capsules en phase huileuse est de 52% par poids après extraction.

La quantité de précurseur hydrolysé est de 4,22g. La quantité de phase huileuse est 4,22g le ratio poids phase huileuse / précurseur de silice est 1.

### Exemple 6

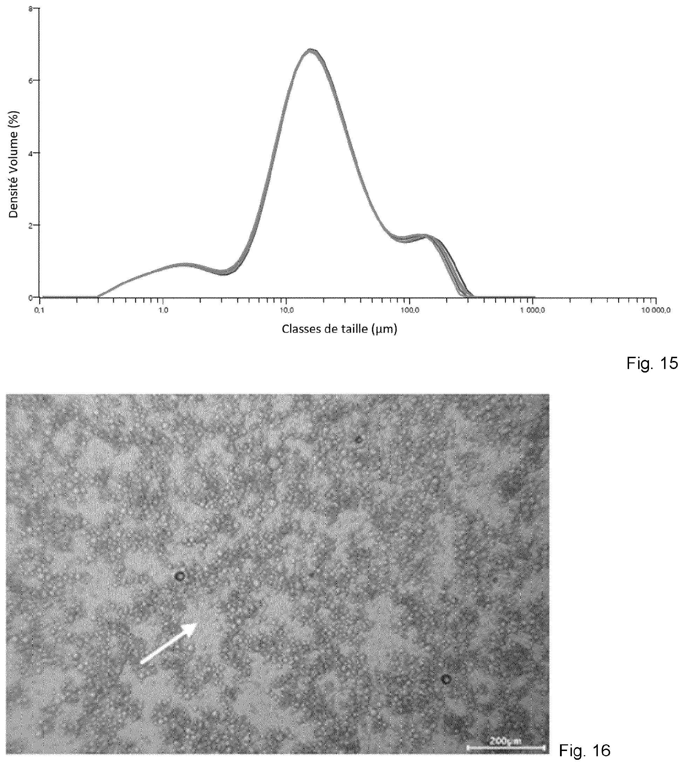

17.3 g de composition parfumée (1) dans 5.8 g miglyol sont émulsionnés dans 23.2 g d'eau avec un mélangeur à fort cisaillement (Turrax T25). Sous cisaillement, 8.0 g de précurseur MTEOS préalablement hydrolysé en conditions acides catalytiques (pH 1.5) sont mélangés à l'émulsion jusqu'à obtenir la taille d'émulsion souhaitée, puis l'émulsion est traitée par 42.1 g de solution aqueuse contenant 1.7 g de polyéthylène imine 25kDa et 3.1 g de sels de phosphate de sodium. Les capsules sont formées instantanément et présentent des tailles entre 1-50µm suivant la force de cisaillement et la géométrie de l'outil de dispersion utilisées. Après 5 minutes, 0.45 g d'APTES sont ajoutés au mélange sous homogénéisation. La distribution granulométrique est mesurée par granulométrie laser (Malvern Mastersizer 3000E, Figure 15). Les capsules sont séparées par centrifugation. 21.2 grammes de microcapsules sont obtenus. Le contenu des capsules en phase huileuse est de 86% par poids après extraction.

La quantité de précurseur hydrolysé est de 4,22g. La quantité de phase huileuse est 23,1g le ratio poids phase huileuse / précurseur de silice est 5,47.

### Exemple 7

### Impact du pH sur la taille des particules obtenues

2.2 g de composition parfumée (2) dans 4.1 g d'huile de ricin sont émulsionnés dans 54.2 g d'eau avec un mélangeur à fort cisaillement (Turrax T25). Sous cisaillement, 8.0 g de précurseur MTEOS préalablement hydrolysé en conditions acides catalytiques (pH 0.5 ou 1.6,) sont mélangés à l'émulsion jusqu'à obtenir la taille d'émulsion souhaitée, puis l'émulsion est traitée par 28.1 g de solution aqueuse contenant 1.1 g de polyéthylène imine 25kDa et 3.1 g de sels de phosphate de sodium. Les capsules sont formées instantanément et présentent des tailles entre 1-100µm suivant la force de cisaillement et la géométrie de l'outil de dispersion utilisées. La distribution granulométrique est mesurée par granulométrie laser (Malvern Mastersizer 3000E). La distribution granulométrique moyenne est réduite de 29µm à 11.5µm par l'augmentation du pH. Après 5 minutes, 0.45 mL de APTES sont ajoutés au mélange sous homogénéisation. Les capsules sont séparées par centrifugation. 8-9.0 grammes de microcapsules sont obtenus. Le contenu des capsules en phase huileuse est de 60% par poids après extraction.

La quantité de précurseur hydrolysé est de 4,22g. La quantité de phase huileuse est 6,3g le ratio poids phase huileuse / précurseur de silice est 1,49.

### Exemple 8

4.4 g de HE Pélargonium dans 8.2 g miglyol 812 sont émulsionnés dans 51.3 g d'eau avec un mélangeur à fort cisaillement (Turrax T25). Sous cisaillement, 6.4ml de précurseur MTEOS préalablement hydrolysé en conditions acides catalytiques (pH=1,6) sont mélangés à l'émulsion jusqu'à obtenir la taille d'émulsion souhaitée, puis l'émulsion est traitée par 28.0 g de solution aqueuse contenant 1.1 g de polyéthylène imine 25kDa. Les capsules sont formées instantanément et présentent des tailles entre 1-50µm suivant la force de cisaillement et la géométrie de l'outil de dispersion utilisées. La morphologie des particules est déterminée par microscopie optique. Les particules ont une morphologie sphérique et une structure de type cœur/écorce et sont observées en présence de gel favorisant l'agrégation des particules (Figure 16). Les capsules sont séparées par centrifugation. 10.0 grammes de microcapsules de HE Pélargonium sont obtenus. Le contenu des capsules en phase huileuse est de 88.5% par poids après extraction.

La quantité de précurseur hydrolysé est de 5,7g. La quantité de phase huileuse est 12,6g le ratio poids phase huileuse / précurseur de silice est 4,16.

### Exemple 9

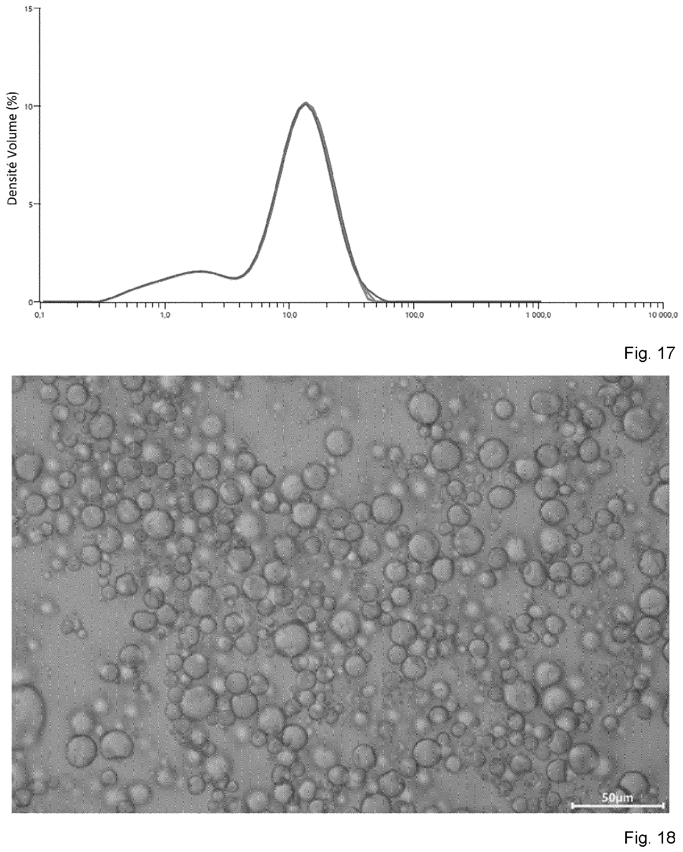

6.7 g de précurseur TEOS sont ajoutés à 3.8 g de composition parfumée (1), 2.0 g de miglyol 812 et 0.85 g d'eau pH=0.5 pendant 15 minutes. Le mélange obtenu est mélangé à 62.5 g d'eau sous cisaillement fort pour former une émulsion jusqu'à la taille souhaitée, puis l'émulsion est traitée par 23.6 g de solution aqueuse contenant 0.8 g de polyéthylène imine 25kDa et 2.1 g de sels de phosphate de sodium. Les capsules sont formées instantanément et présentent des tailles entre 1-50µm suivant la force de cisaillement et la géométrie de l'outil de dispersion utilisées. La distribution granulométrique est mesurée par granulométrie laser (Malvern Mastersizer 3000E, Figure 17). La morphologie des particules est déterminée par microscopie optique. Les particules ont une morphologie sphérique et une structure de type cœur/écorce (Figure 18). Les capsules sont séparées par centrifugation. 3.4 grammes de microcapsules sont obtenus. Le contenu des capsules en phase huileuse est de 72.6% par poids après extraction.

La quantité de précurseur hydrolysé est de 3,09g. La quantité de phase huileuse est 5,8g le ratio poids phase huileuse / précurseur de silice est 1,88.

### Exemple 10

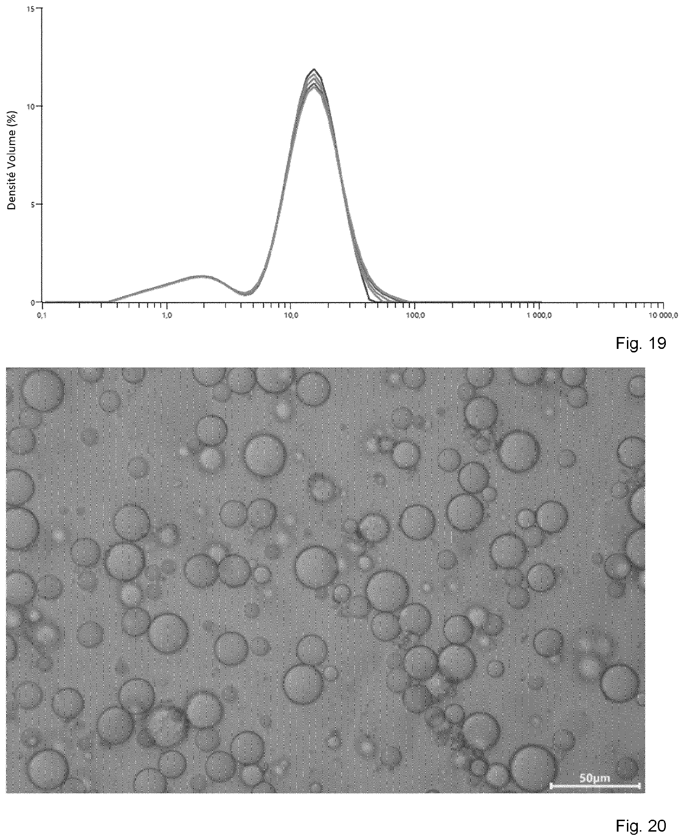

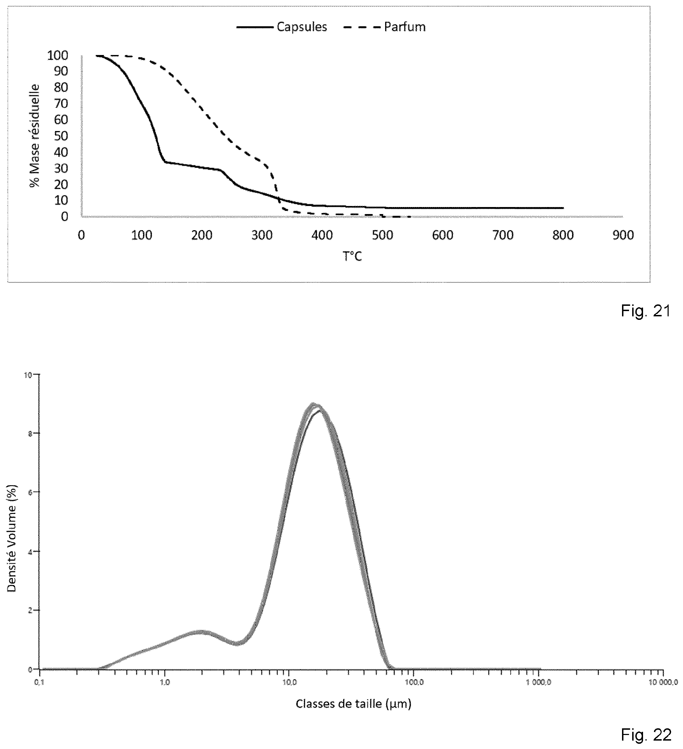

6.7 g de précurseur TEOS sont ajoutés à 2.3 g de composition parfumée (1), 3.5 g de miglyol 812 et 0.85 g d'eau pH =0.5 pendant 15 minutes. Le mélange obtenu est mélangé à 62.5 g d'eau sous cisaillement fort pour former une émulsion jusqu'à la taille souhaitée, puis l'émulsion est traitée par 23.6 g de solution aqueuse contenant 0.8 g de polyéthylène imine 25kDa et 2.1 g de sels de phosphate de sodium Les capsules sont formées instantanément et présentent des tailles entre 1-50µm suivant la force de cisaillement et la géométrie de l'outil de dispersion utilisées. La distribution granulométrique est mesurée par granulométrie laser (Malvern Mastersizer 3000E, Figure 19). La morphologie des particules est déterminée par microscopie optique. Les particules ont une morphologie sphérique et une structure de type cœur/écorce (Figure 20). Les capsules sont séparées par centrifugation. 4.0 grammes de microcapsules sont obtenus. Le contenu des capsules en phase huileuse parfumée est de 89% par poids par analyse ATG (Mettler Toledo TGA2) sur la base de la perte de masse comprise en 110-400°C (rampe 10°C/min entre 25 et 800°C, Figure 21).

La quantité de précurseur hydrolysé est de 3,09g. La quantité de phase huileuse est 5,8g le ratio poids phase huileuse / précurseur de silice est 1,88.

### Performance olfactive des microcapsules parfumées

Formulation en base avec tensio-actif :
Des capsules obtenues avec l'Exemple 9 et 10, sous forme de suspension dans l'eau, ont été formulées en base nettoyant tout usage (All Purpose Cleaner ou APC) à 0.3% équivalent parfum. Un échantillon de 0.5 mL est appliqué et étalé de manière homogène sur une surface lisse de 8.3cm x 10cm. Le support est laissé sécher à l'air libre 4h à température ambiante puis évalué pas un minimum de 4 panélistes. L'intensité du parfum est notée de 0 à 10. Une valeur de 5 indique que le parfum est bien détectable avec une intensité moyenne et une intensité de 10 indique une intensité très forte. Après 4h, le parfum est détecté et son odeur décrite précisément.

Après frottement de la surface au moyen d'un essuyeur de précision, une intensité plus forte est obtenue. L'intensité est encore plus forte pour le produit contenant le parfum dans des capsules indiquant que les capsules sont capables de retenir efficacement une composition parfumée et capable de délivrer une expérience améliorée d'un produit parfumé.

**[Table 2]**

| | Avant frottement | Après frottement |
|---|---|---|
| Exemple 9 | 3-4 | +3 |
| Exemple 10 | 3-4 | +2 |
| Parfum libre | 3-4 | 0 |

Formulation en base avec tensio-actif dans une application rincée (shampoing) :
Des capsules obtenues à l'exemple 9, sous forme de suspension dans l'eau, ont été formulées en base shampoing à 0.4% parfum. Une application de 1.5g de shampoing sur une mèche de cheveux (7g) est réalisée puis la mèche est rincée sous l'eau tiède. L'application est réalisée deux fois. La mèche est ensuite essorée et séchée à l'air libre 5h puis évaluée pas un minimum de 4 panélistes. L'intensité du parfum est notée de 0 à 10. Une valeur de 5 indique que le parfum est bien détectable avec une intensité moyenne et une intensité de 10 indique une intensité très forte. Après 5h, le parfum est détecté et son odeur décrite précisément (intensité 3-4). Après frottement de la mèche avec les doigts, une intensité plus forte (+2) est obtenue indiquant que les capsules sont capables de retenir efficacement une composition parfumée et capable de délivrer une expérience améliorée d'un produit parfumé rincé.

### Exemple 11

6.7 g de précurseur TEOS sont ajoutés à 2.3 g de HE Orange Douce, 3.5 g de miglyol 812 et 0.85 g d'eau pH =0.5 pendant 15 minutes. Le mélange obtenu est mélangé à 62.5 g d'eau sous cisaillement fort pour former une émulsion jusqu'à la taille souhaitée, puis l'émulsion est traitée par 23.6 g de solution aqueuse contenant 0.8 g de polyéthylène imine 25kDa et 2.1 g de sels de phosphate de sodium. Les capsules sont formées instantanément et présentent des tailles entre 1-50µm suivant la force de cisaillement et la géométrie de l'outil de dispersion utilisées. La distribution granulométrique est mesurée par granulométrie laser (Malvern Mastersizer 3000E,

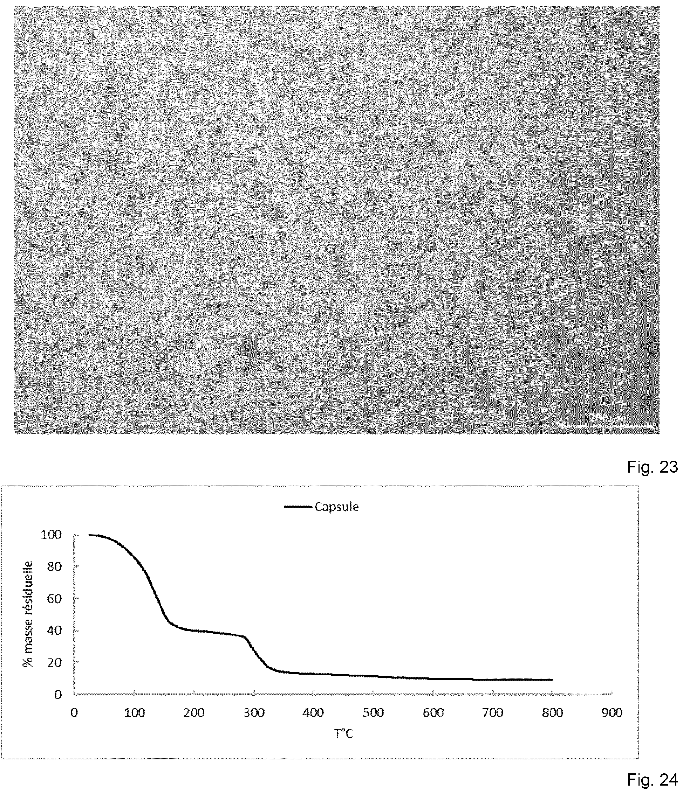

Figure 22). La morphologie des particules est déterminée par microscopie optique. Les particules ont une morphologie sphérique et une structure de type cœur/écorce (Figure 23). Les capsules sont séparées par centrifugation. 4.2 grammes de microcapsules sont obtenus. Le contenu des capsules en phase huileuse est de 84% par poids par analyse ATG (Mettler Toledo TGA2) sur la base de la perte de masse comprise en 110-400°C (rampe 10°C/min entre 25 et 800°C, Figure 24).

La quantité de précurseur hydrolysé est de 3,09g. La quantité de phase huileuse est 5,8g le ratio poids phase huileuse / précurseur de silice est 1,88.

### Exemple 12

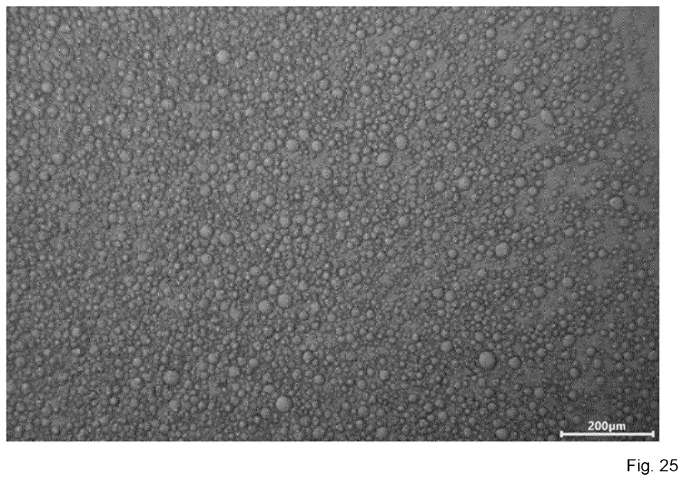

2,3 g de composition parfumée (1) dans 3,5 g de miglyol 812 sont ajoutés à 6,7 g de précurseur TEOS. Le mélange est agité 15 minutes après addition de 0.85 g d'eau pH 0.5. Le mélange obtenu est mélangé à 62,5 g d'eau sous cisaillement fort pour former une émulsion jusqu'à la taille souhaitée, puis l'émulsion est traitée par 23.6 g de solution aqueuse contenant 0.8 g de polyéthylène imine 25kDa et 2.1 g de sels de phosphate de sodium. Les capsules sont formées instantanément et présentent des tailles entre 1-50µm suivant la force de cisaillement et la géométrie de l'outil de dispersion utilisées. La morphologie des particules est déterminée par microscopie optique. Les particules ont une morphologie sphérique et une structure de type cœur/écorce (Figure 25). Les capsules sont séparées par centrifugation. 4.0 grammes de microcapsules sont obtenus. Le contenu des capsules en phase huileuse est de 84% par poids par extraction.

La quantité de précurseur hydrolysé est de 3,09g. La quantité de phase huileuse est 5,8g le ratio poids phase huileuse / précurseur de silice est 1,88.

### Exemple 13

Les paramètres des exemples du procédé du document FR 3 112 494 utilisant un actif lipophile sont présentés ci-après.

Dans tous les exemples le précurseur de silice est le MTEOS.

La quantité de précurseur utilisé pour le calcul du ratio est la quantité de précurseur Si sous forme hydrolysé. C'est le précurseur hydrolysé qui va réagir et qui est donc pris en considération dans le ratio.

Cette quantité de précurseur de silice sous forme hydrolysé est égale à (quantité de précurseur / Masse moléculaire précurseur) x Masse moléculaire du précurseur de silice hydrolysé.

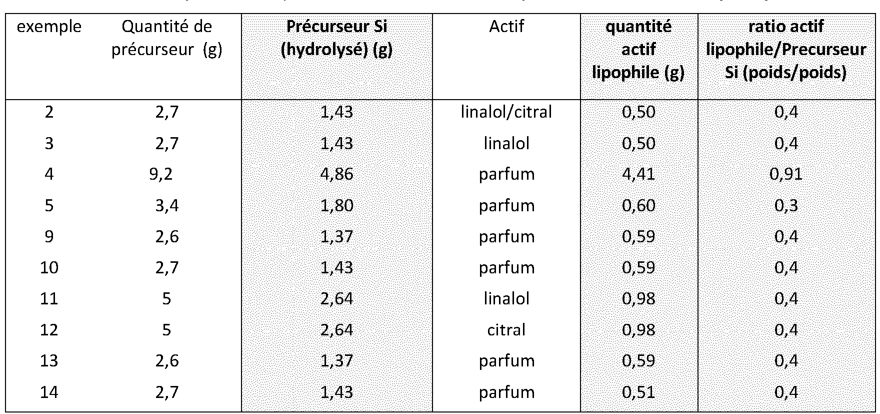

On constate que ce document ne décrit pas de ratio selon l'invention, c'est-à-dire en faveur de la phase huileuse mais bien à l'inverse en faveur du précurseur de silice.

## Revendications

1. Procédé de synthèse de microcapsules de silice comprenant une phase huileuse comprenant au moins un actif lipophile comprenant les étapes suivantes :
a) préparation de nucléi de silice par hydrolyse d'au moins un précurseur de silice dans l'eau en milieu catalytique acide, préférentiellement la quantité d'acide est choisie de sorte à être inférieure ou égale à 0,05 équivalent d'acide par rapport au précurseur de silice,
a') ajout à l'issu de l'étape a) ou au cours de l'étape a) d'une phase huileuse dans un ratio en poids phase huileuse/ précurseur de silice supérieur ou égal à 1/1, préférentiellement inférieur ou égal à 7/1 .
b) formation d'une phase matrice par mélange d'au moins un agent de condensation choisi parmi au moins un polymère polycationique basique branché ou linéaire choisi parmi l'un au moins parmi les polyéthylènes imines, les polyaminoacides, les polyallylamines, des dérivés des propylènes imines, les polylysines, les dérivés polysaccharides à galactomannanes, fructo-oligosaccharides et oligofructoses ou un mélange, et d'au moins un anion monovalent, divalent ou trivalent choisi parmi l'un au moins parmi un sel de phosphate, un sel de tartrate et un sel de citrate, un sel de sulfate, ou un sel de nitrate,
c) condensation en milieu basique des nucléis de silice obtenus à l'étape a) et de la phase huileuse par mélange sous agitation avec la phase matrice obtenue à l'étape b) à pH alcalin inférieur ou égal à 10.

2. Procédé selon la revendication précédente dans lequel la phase huileuse est mise en émulsion avec de l'eau avant son ajout à l'issu de l'étape a).

3. Procédé selon la revendication 1 dans lequel la phase huileuse est mélangée au milieu catalytique acide avant son mélange avec l'au moins un précurseur de silice, puis avec de l'eau pour former une émulsion.

4. Procédé selon la revendication 1 dans lequel la phase huileuse est mélangée au milieu catalytique acide et à l'au moins un précurseur de silice avant d'être mélangée à de l'eau pour former une émulsion.

5. Procédé selon l'une quelconque des revendications précédentes dans lequel la phase huileuse comprend au moins un actif lipophile volatil.

6. Procédé selon l'une quelconque des revendications 1 à 4 dans lequel la phase huileuse comprend au moins un actif lipophile non volatil.

7. Procédé selon l'une quelconque des revendications précédentes dans lequel la phase huileuse comprend un solvant huileux ou un mélange de solvants huileux.

8. Procédé selon l'une quelconque des revendications précédentes dans lequel le ratio molaire polymères cationiques basiques / monomères de silice issus du précurseur de silice est supérieur ou égal à 0,3 et inférieur ou égal à 1.

9. Procédé selon l'une quelconque des revendications précédentes comprenant une étape c'), postérieure ou simultanée à l'étape c) de condensation, comprenant une étape de fonctionnalisation des capsules.

10. Procédé selon la revendication précédente dans lequel l'étape de fonctionnalisation des capsules comprend l'ajout d'au moins un précurseur de silice.

11. Procédé selon la revendication précédente dans lequel le précurseur de silice pour la fonctionnalisation représente maximum 10%, préférentiellement 7% en poids du poids total du précurseur de silice utilisé à l'étape a), préférentiellement la fonctionnalisation représente un poids inférieur ou égale à 5% du poids total du précurseur de silice utilisé à l'étape a).

12. Procédé selon l'une quelconque des revendications précédentes dans lequel l'agent de condensation est ajouté à l'étape b) pour être en quantité inférieure ou égale à 100g/L dans la phase matrice, préférentiellement inférieure ou égale à 50g/L, préférentiellement inférieure ou égale à 20g/L.

13. Procédé selon l'une quelconque des revendications précédentes dans lequel le précurseur de silice ou un mélange de précurseur de l'étape a) est choisi parmi l'un au moins parmi orthosilicate de tétraéthyle (TEOS), orthosilicate de tétraméthyle (TMOS), (3-Aminopropyl)triéthoxysilane (APTES), metasilicate de sodium, calcium silicate, triméthoxyméthylsilane (MTMOS), triéthoxyméthylsilane (MTEOS), triéthoxysilane, triméthoxysilane, triéthoxy(éthyl)silane (ETEOS), triméthoxy(éthyl)silane (ETEOS), isobutyl(triméthoxy)silane, propyl(triméthoxy)silane, orthosilicate de sodium.

14. Procédé selon l'une quelconque des revendications 1 à 12 dans lequel le précurseur de silice de l'étape a) est choisi parmi l'un au moins parmi un extrait de silice biogénique, du sodium silicate ou une source naturelle d'acide ortho silicique.

15. Procédé selon l'une quelconque des revendications précédentes dans lequel le au moins un précurseur de silice est ajouté à l'étape a) en quantité inférieure ou égale à 6000mM, préférentiellement le au moins un précurseur de silice est présent à l'étape c) en quantité inférieure ou égale à 1000mM.

16. Procédé selon l'une quelconque des revendications précédentes dans lequel l'anion monovalent, divalent ou trivalent est ajouté en concentration inférieure ou égale à 300mM.

17. Procédé selon l'une quelconque des revendications 1 à 16 dans lequel le mélange de l'étape b) est ajouté aux nucléis obtenus à l'étape a).

18. Procédé selon l'une quelconque des revendications précédentes comprenant après l'étape c) une étape d) de séparation des capsules par centrifugation ou filtration ou décantation et éventuellement une étape e) de lavage des capsules isolées et éventuellement une étape f) de séchage des capsules.
